# EUROPEAN PATENT SPECIFICATION

(11) **EP 3 653 599 B1**
(45) Date of publication and mention of the grant of the patent: **14.12.2022**
(21) Application number: 19208228.7
(22) Date of filing: 18.04.2017
(51) Int. Cl.: C07C 215/54, A61K 31/137, A61P 29/00

(54) **CRYSTALLINE TAPENTADOL PHOSPHATE**
KRISTALLINES TAPENTADOLPHOSPHAT
PHOSPHATE TAPENTADOL CRISTALLIN

(30) Priority: 19.04.2016 EP 16166099; 19.04.2016 EP 16166101; 23.05.2016 EP 16170780; 23.05.2016 EP 16170781
(43) Date of publication of application: 20.05.2020
(62) Divisional of application: 17716933.1
(73) Proprietor: ratiopharm GmbH, 89079 Ulm (DE)
(72) Inventor: ALBRECHT, Wolfgang, 89075 Ulm (DE); GUSERLE, Richard, 89259 Kötz (DE); GEIER, Jens, 72534 Hayingen (DE)
(74) Representative: Ter Meer Steinmeister & Partner

(56) References cited:
- WO-A1-2012/051246

## Description

### Background of the invention

The present invention relates to the use of tapentadol phosphate in crystalline form for preparing a pharmaceutical composition and to a method for the preparation of a composition by specifically processing crystalline tapentadol phosphate and to the composition obtainable by said specifically processing crystalline tapentadol phosphate.

The IUPAC name of tapentadol is 3-(1*R*,2*R*)-(3-dimethylamino)-1-ethyl-2-methylpropyl]phenol. Tapentadol is represented by the following chemical structure according to Formula (I):

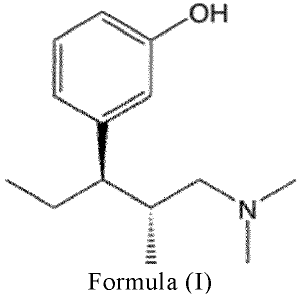

Tapentadol is an analgesic whose effect is reported to be based on two molecular mechanisms. Firstly, like opioids, tapentadol may activate µ-receptors and thus presynaptically and postsynaptically attenuates the transmission of pain stimuli in the spinal cord and brain. Secondly, tapentadol may act as a noradrenalin re-uptake inhibitor and thus increases the concentration of that nerve messenger in the synaptic gap.

Synthesis pathways for tapentadol and its use as an analgesic are described in EP 0 693 475 A1.

Further, tapentadol is reported to be present in form of addition salts, wherein for example WO 2006/000441 A2 describes tapentadol in form of its hydrochloride.

Further, WO 2012/010316 A1 relates to salts or co-crystals of tapentadol with at least one acid component. Said document refers to a long list of acid components, wherein among many others phosphoric acid is mentioned. However, no further information is given, neither to its method of preparation nor to its properties. Thus, the alleged tapentadol phosphate cannot be regarded as being enabled by the above-mentioned application.

WO 2012/051246 A1 substantially relates to the preparation of tapentadol hydrobromide. Further, in Example 8 said document inter alia describes the reaction of tapentadol free base with phosphoric acid, which resulted in tapentadol phosphate. The phosphate addition salt can be obtained as solid, which, however, is reported to liquefy within a few seconds. With respect to the workability and the regulatory requirements for pharmaceutical compositions and dosage forms such behaviour of the active pharmaceutical ingredient is highly undesirable.

Hence, it was an object of the present invention to overcome the above drawbacks.

In particular, tapentadol phosphate should be provided in a form that shows improved properties with regard to processability. In particular, tapentadol phosphate should be provided in a form which allows its use in the formation of a pharmaceutical composition which fulfils the regulatory requirements and which can be easily converted into a dosage form without the use of complex and cost-intensive methods.

All the above-mentioned objectives should preferably be solved for tapentadol phosphate designed for a dosage form with immediate release ("IR") and for modified release ("MR").

### Summary of the Invention

According to the present invention, the above objectives can be achieved by the use of crystalline tapentadol phosphate for the preparation of a pharmaceutical composition or a dosage form.

Thus, the subject of the invention is the subject matter as defined in claim 1.

In the context of this invention, the term "tapentadol phosphate" refers to phosphoric acid addition salt of tapentadol according to Formula (II)

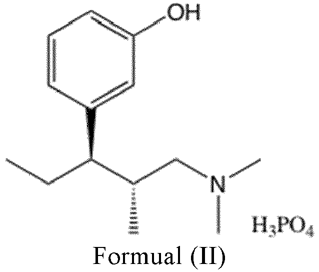

In addition, the term "tapentadol phosphate" as used in the present application can refer to tapentadol phosphate hydrates, solvates, polymorphs and mixtures thereof.

The tapentadol phosphate is in crystalline form.

In a preferred embodiment the crystalline tapentadol phosphate is crystalline tapentadol phosphate having a water content of 0.5 to 4.0%, preferably 1.0 to 3.5%, in particular 1.5 to 3.0%, wherein the water content is determined by thermogravimetry. In the present application, the thermogravimetry is carried out as described below in the experimental section.

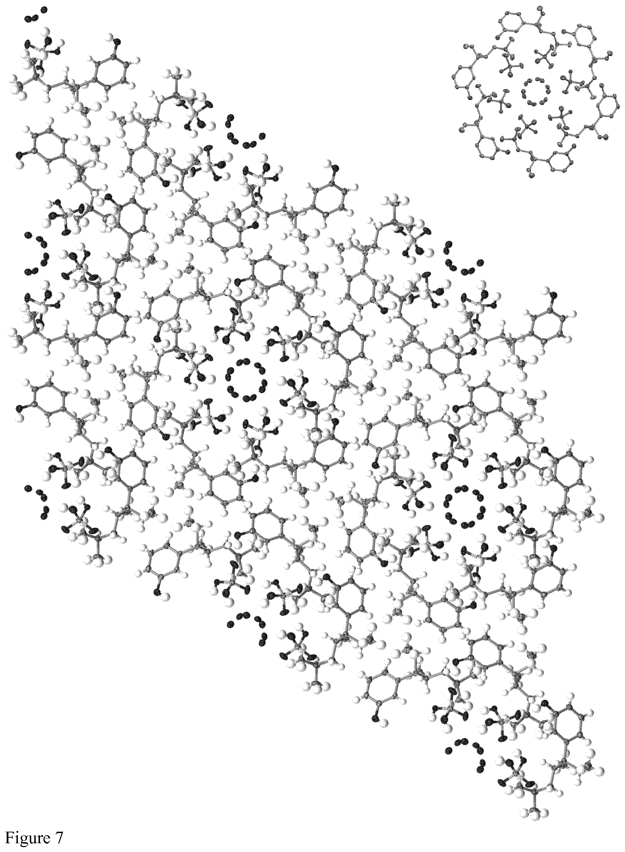

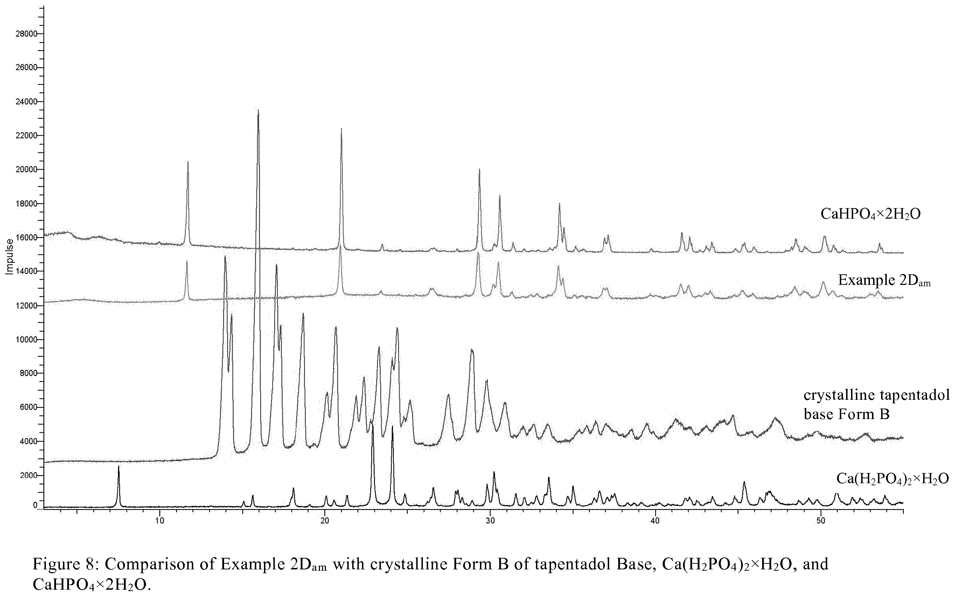

Figure 7 shows the molecular structure of crystalline tapentadol phosphate. As can be seen from this Figure, the molecular structure of crystalline tapentadol phosphate contains channels in which water molecules can migrate and through which water molecules can diffuse in or out.

A crystal form may be referred to herein as being characterized by data selected from two or more different data groupings, for example by a powder XRD pattern, having a group of specific peaks or by a powder XRD pattern as depicted in a diffractogram, or by "a combination thereof' (or "combinations thereof' or "any combination thereof'). These expressions, e.g. "any combination thereof', contemplate that the skilled person may characterize a crystal form using any combination of the recited characteristic analytical data. For example, the skilled person may characterize a crystal form using a group of three, four or five characteristic powder XRD peaks and supplement this characterization with one or more additional feature(s) observed in the powder X-ray diffractogram, e.g. an additional peak, a characteristic peak shape, a peak intensity or even the absence of a peak at some position in the powder XRD pattern. Alternatively, the skilled person may in some instances characterize a crystal form using a group of three, four or five characteristic powder XRD peaks.

In the present application, the XRPD is measured as described below in the experimental section. Further, unless indicated otherwise, XRPD peaks are reported as degrees 2θ values with a standard error of ± 0.2 degrees 2θ.

A crystal form may be referred to herein as being characterized by graphical data "as depicted in" a particular figure. Such data include for example powder X-ray diffractograms. The skilled person will understand that such graphical representation of data may be subject to small variations, e.g. in peak relative intensities and peak positions, due to factors such as variations in instrument response and variations in sample preparation, which are well-known to the skilled person. Nonetheless, the skilled person would readily be capable of comparing the graphical data in the figures herein with graphical data generated for an unknown crystal form, and confirm as to whether the two sets of graphical data characterize the same crystal form or two different crystal forms.

In a preferred embodiment crystalline tapentadol phosphate can preferably have characteristic X-ray powder diffraction peaks at 5.1, 14.4, 17.7, 18.3 and 21.0 degrees 2θ (± 0.2 degrees 2θ).

In a preferred embodiment crystalline tapentadol phosphate can be characterized by one or more further XRPD diffraction peak(s) at 8.7, 17.6, 20.3, 22.1 and/or 23.4 degrees 2θ (± 0.2 degrees 2θ). This tapentadol phosphate can preferably refer to tapentadol phosphate in type A.

In an alternatively further preferred embodiment of the present invention crystalline tapentadol phosphate can be characterized by the XRPD diffraction peak(s) at degrees 2θ ± 0.2 degrees 2θ (intensity %): 5.1 (100), 8.7 (10), 10.1 (1), 12.5 (3), 13.4 (4), 14.4 (37), 15.3 (4), 17.6 (12), 17.7 (15), 18.3 (27), 19.1 (7), 20.3 (11), 21.0 (25), 21.6 (9), 22.1 (18), 23.4 (14), 24.8 (7), 25.0 (17), 25.3 (12), 26.0 (9), 26.5 (7), 26.8 (9), 28.0 (5), 28.4 (4), 28.9 (10), 29.2 (6), 29.4 (4), and 29.6 (2).

In an alternatively further preferred embodiment of the present invention crystalline tapentadol phosphate can be characterized by the XRPD diffraction peak(s) at degrees 2θ ± 0.2 degrees 2θ: 5.1, 8.7, 10.1, 12.5, 13.4, 14.4, 15.3, 17.6, 17.7, 18.3, 19.1, 20.3, 21.0, 21.6, 22.1, 23.4, 24.8, 25.0 and 25.3.

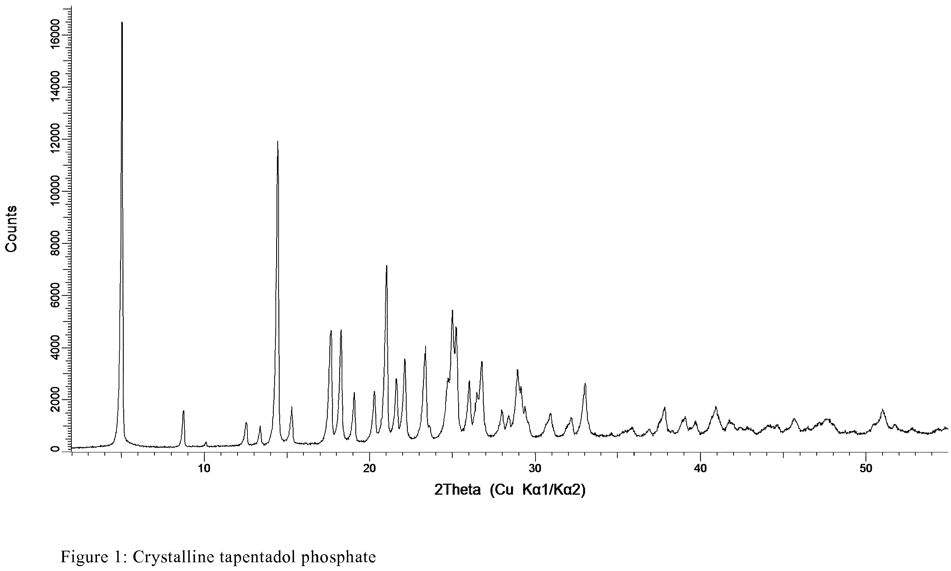

An XRPD diffraction pattern of a preferred embodiment of the crystalline tapentadol phosphate is shown in Figure 1.

The crystalline tapentadol phosphate may consist of crystalline tapentadol phosphate. Alternatively, it may also contain small amounts of amorphous tapentadol phosphate components. A mixture containing 60 to 99.999% by weight crystalline tapentadol phosphate and 0.001 to 40% by weight amorphous tapentadol phosphate is preferred, more preferably 90 to 99.99% by weight crystalline tapentadol phosphate and 0.01 to 10% amorphous tapentadol phosphate, particularly preferably 95 to 99.9% by weight crystalline tapentadol phosphate and 0.1 to 5% amorphous tapentadol phosphate.

In a preferred embodiment of the invention the tapentadol phosphate is present in combination with MHPO₄ or M'₂HPO₄, wherein M is a divalent cation and M' is a monovalent cation.

MHPO₄ or M'₂HPO₄, with M being a divalent cation and M' being a monovalent cation, can also refer to hydrates of MHPO₄ or M'₂HPO₄

In a preferred embodiment the tapentadol phosphate is present in combination with MHPO₄, wherein M is a divalent cation. In a preferred embodiment divalent cation M' can be a cation of an alkaline earth metal. Examples of alkaline earth metals are magnesium or calcium. Especially preferred is calcium.

In a preferred embodiment the monovalent cation M' can be a cation of an alkaline metal. Examples of alkaline metals are lithium, sodium and potassium, in particular sodium.

In a preferred embodiment the crystalline tapentadol phosphate is present in solid form at 23°C. This includes that the crystalline tapentadol phosphate does not liquefy or is not present in a solution.

In a further embodiment crystalline tapentadol phosphate can be present in an isolated form. In the context of the invention an isolated form of crystalline tapentadol phosphate can be referred to as crystalline tapentadol phosphate being in an essentially pure form. In particular, the crystalline tapentadol phosphate can preferably not be present in combination with a carrier for example in form of a solid solution.

In an alternatively preferred embodiment crystalline tapentadol phosphate can preferably have characteristic X-ray powder diffraction peaks at 5.1, 14.3, 17.6, 18.5 and 21.1 degrees 2θ (± 0.2 degrees 2θ).

In a preferred embodiment crystalline tapentadol phosphate can be characterized by one or more further XRPD diffraction peak(s) at 8.9, 20.5, 22.4, 23.5 and/or 24.3 degrees 2θ (± 0.2 degrees 2θ). This tapentadol phosphate can preferably refer to tapentadol phosphate in type B.

In an alternatively further preferred embodiment of the present invention crystalline tapentadol phosphate can be characterized by the XRPD diffraction peak(s) at degrees 2θ ± 0.2 degrees 2θ (intensity %): 5.1 (100), 8.9 (3), 12.4 (5), 13.5 (4), 14.3 (42), 15.2 (4), 17.6 (21), 18.5 (18), 19.1 (7), 20.5 (14), 21.1 (28), 21.7 (9), 22.4 (14), 23.5 (15), 24.3 (6), 24.9 (19), 25.1 (16), 25.8 (3), 26.2 (12), 26.4 (13), 26.7 (5), 27.3 (2), 28.2 (5), 28.8 (8), 29.1 (11), 29.4 (5), and 29.6 (5).

In an alternatively further preferred embodiment of the present invention crystalline tapentadol phosphate can be characterized by the XRPD diffraction peak(s) at degrees 2θ ± 0.2 degrees 2θ: 5.1, 8.9, 12.4, 13.5, 14.3, 15.2, 17.6, 18.5, 19.1, 20.5, 21.1, 21.7, 22.4, 23.5, 24.3, 24.9, and 25.1.

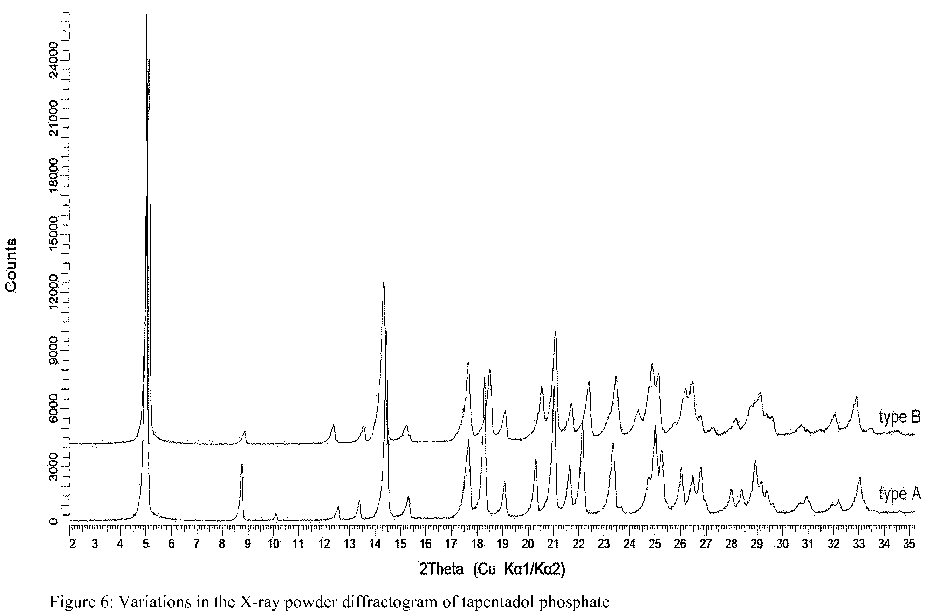

The differences in the diffractograms of type A and type B of tapentadol phosphate can be best recognized by a direct graphical comparison (Fig. 6).

Type B can for example produced from type A by heating at 70°C under a pressure of 8 mbar.

Described herein is a method of preparing crystalline tapentadol phosphate comprising the steps of
(a) providing tapentadol and phosphoric acid
(b) milling the mixture from step (a)

In a preferred embodiment in step (a) tapentadol and phosphoric acid can be provided. Tapentadol is preferably provided in form of its free base. In a particularly preferred embodiment tapentadol free base in polymorphic form B is provided. Form B of tapentadol free base can be prepared as described for example in WO 2009/071310.

Phosphoric acid can preferably be provided in form of 85% phosphoric acid.

In a further preferred embodiment of the invention in step (a) the molar ratio of tapentadol to phosphoric acid is from 3:1 to 1:1.5, preferably from 2.5 to 1:1.4, more preferably from 2:1 to 1:1.2, in particular about 1:1.

Further, step (a) can comprise mixing of tapentadol and phosphoric acid.

In a further preferred embodiment of the method of the present invention in step (a) or in step (b) water is added, wherein the weight ratio of tapentadol to water is from 50:1 to 500:1, preferably from 75:1 to 400:1.

In step (b) the mixture from step (a) can preferably be milled. The milling can preferably be performed in conventional milling apparatuses, such as in a ball mill, air jet mill, pin mill, classifier mill, cross beater mill, disk mill, mortar grinder or a rotor mill. A ball mill is preferably used. It is further preferred that 2 to 10 balls, preferably 3 to 7 balls, are used.

The milling time is preferably 0.5 minutes to 90 minutes, preferably 2 to 60 minutes, more preferably 5 to 30 minutes, in particular about 15 minutes.

It is preferred that the milled mixture can be sieved, preferably with a sieve having a mesh size of 1 to 1000 µm, preferably 20 to 800 µm, especially 25 to 600 µm.

An alternative subject of the present invention is a method of preparing tapentadol phosphate comprising the steps of
(a') providing tapentadol and M(H₂PO₄)₂ or M'H₂PO₄
(b') milling the mixture from step (a)

In a preferred embodiment in step (a') tapentadol and M(H₂PO₄)₂ or M'H₂PO₄ can be provided. Tapentadol is preferably provided in form of its free base. In a particularly preferred embodiment tapentadol free base in polymorphic form B is provided.

Generally, the comments made above for M or M' can also apply to the above described method.

In the method M(H₂PO₄)₂ or M'H₂PO₄ can be present in the form of the corresponding hydrate. In a particularly preferred embodiment M'H₂PO₄ refers to NaH₂PO₄x2H₂O.

In a particularly preferred embodiment in step (a) tapentadol free base in polymorphic form B and NaH₂PO₄x2H₂O are used.

Further, step (a') can comprise mixing of tapentadol and M(H₂PO₄)₂ or M'(H₂PO₄).

In a further preferred embodiment of the method of the present invention in step (a') or in step (b') water is added, wherein the weight ratio of tapentadol and water is from 50:1 to 500:1, preferably from 75:1 to 400:1.

In step (b') the mixture from step (a') can preferably be milled. The milling can preferably be performed in conventional milling apparatuses, such as in a ball mill, air jet mill, pin mill, classifier mill, cross beater mill, disk mill, mortar grinder or a rotor mill. A ball mill is preferably used. It is further preferred that 2 to 10 balls, preferably 3 to 7 balls, are used.

The milling time is preferably 0.5 minutes to 90 minutes, preferably 2 to 60 minutes, more preferably 5 to 30 minutes, in particular about 15 minutes.

It is preferred that the milled mixture can be sieved, preferably with a sieve having a mesh size of 1 to 1000 µm, preferably 20 to 800 µm, especially 25 to 600 µm.

Described herein is an alternative method of preparing tapentadol phosphate comprising the steps of
(a') providing tapentadol and M(H₂PO₄)₂ or M'(H₂PO₄)
(b') milling the mixture from step (a)
(c') subjecting the product from step (b') to an DVS treatment

In a preferred embodiment in step (a') tapentadol and M(H₂PO₄)₂ or M'(H₂PO₄) can be provided. Tapentadol is preferably provided in form of its free base. In a particularly preferred embodiment tapentadol free base in polymorphic form B is provided.

Generally, the comments made above for M or M' can also apply to the method of the present invention.

In the method M(H₂PO₄)₂ or M'(H₂PO₄) can be present in the form of the corresponding hydrate. In a particularly preferred embodiment M(H₂PO₄)₂ refers to Ca(H₂PO₄)₂xH₂O.

In a particularly preferred embodiment in step (a') tapentadol free base in polymorphic form B and Ca(H₂PO₄)₂xH₂O are used.

Further, step (a') can comprise mixing tapentadol and M(H₂PO₄)₂ or M'(H₂PO₄).

In the method in step (a') or in step (b') water is added, wherein the weight ratio of tapentadol and water is from 50:1 to 500:1, preferably from 75:1 to 400:1.

In step (b') the mixture from step (a') can preferably be milled. The milling can preferably be performed in conventional milling apparatuses, such as in a ball mill, air jet mill, pin mill, classifier mill, cross beater mill, disk mill, mortar grinder or a rotor mill. A ball mill is preferably used. It is further preferred that 2 to 10 balls, preferably 3 to 7 balls, are used.

The milling time is preferably 0.5 minutes to 90 minutes, preferably 2 to 60 minutes, more preferably 5 to 30 minutes, in particular about 15 minutes.

It is preferred that the milled mixture can be sieved, preferably with a sieve having a mesh size of 1 to 1000 µm, preferably 20 to 800 µm, especially 25 to 600 µm.

In step (c') the product from step (b') can be subjected to a DVS treatment. In the present application, parameters for a DVS treatment can be found in the experimental section as described below.

Described herein is an alternative method of preparing tapentadol phosphate comprising the steps of
(a") providing a mixture of tapentadol and phosphoric acid in a solvent,
(b") seeding the mixture from step (a") with crystalline tapentadol phosphate,
(c") isolating crystalline tapentadol phosphate.

In step (a") a mixture of tapentadol and phosphoric acid in a solvent is provided. The solvent can be an organic solvent. Examples of solvents are methanol, ethanol, isopropanol, acetone, dioxane, tetrahydrofuran, acetonitrile and mixtures thereof. Ethanol is preferred.

Further, the solvent can preferably contain water, wherein the content of water can be preferably from 0 to 20% v/v, more preferably from 0.5 to 10% v/v, in particular from 1.0 to 5.0% v/v.

In a particularly preferred embodiment the solvent is a mixture of ethanol and water with 96% v/v of ethanol and 4% v/v of water.

In a further particularly preferred embodiment the solvent is a mixture of ethanol and water with 99% v/v of ethanol and 1% v/v of water.

It is preferred that tapentadol and phosphoric acid are present in a molecular ratio of 0.8:1 to 1.2:1, more preferably 0.9:1 to 1.1:1, in particular 0.95:1 to 1.05:1.

In a preferred embodiment step (a") can comprise the substeps of
(a"I) dissolving or suspending tapentadol in a solvent,
(a"II) adding phosphoric acid to the solution or suspension from step (a"I).

In step (a"I) tapentadol, preferably tapentadol in form of its free base, is dissolved, preferably completely dissolved, in a solvent. Examples of solvents correspond to the ones mentioned above, preferably ethanol/water 96% v/v is used as solvent.

It is further preferred that step (a"I) is carried out under mechanical movement such as stirring.

In step (a"II) phosphoric acid can be preferably added to the solution from step (a"I). It is preferred that the phosphoric acid can be 85% phosphoric acid.

It is further preferred that while adding phosphoric acid to the solution of step (a"I) the solution is subjected to a mechanical movement such as stirring.

In a preferred embodiment step (a"II) includes further stirring for 1 minute to 1 hour, preferably for 5 minutes to 30 minutes, in particular for 10 to 20 minutes. Further, the stirring can be carried out at temperatures of 10 to 60°C, preferably 15 to 55°C, in particular 20 to 25°C.

In step (b") the mixture from step (a"), preferably in form of a solution, can be seeded with crystalline tapentadol phosphate. Crystalline tapentadol phosphate for seeding can for example be obtained as described above. It is further preferred that while adding seeds of tapentadol phosphate to the mixture of step (a"II), preferably in from of a solution, the mixture of step (a"II) is subjected to a mechanical movement such as stirring.

Step (c") of isolating crystalline tapentadol phosphate can preferably comprise cooling the mixture of step (b"), preferably to a temperature of 0°C to 27°C, more preferably 5°C to 25°C, in particular 15°C to 25°C.

Further, in step (c") the isolating of crystalline tapentadol phosphate can preferably be carried out by filtering off the solid. Filtering off can be preferably conducted by a vacuum filtration. Further, crystalline tapentadol phosphate can preferably be washed, preferably with a solvent as mentioned above, in particular with a solvent as mentioned above at a temperature of 0 to 20°C, preferably 5 to 15°C. Subsequently, the crystalline tapentadol phosphate can preferably be dried. Drying can preferably be carried out at a temperature of 23°C to 70°C, preferably 30°C to 60°C. Alternatively or additionally, the drying can preferably be carried out under reduced pressure of 1 to 500 mbar, in particular 3 to 45 mbar.

Pharmaceutically acceptable excipient(s) can for example be fillers, binders, glidants, disintegrants, lubricants, flow regulating agents and release agents. Suitable excipients are for example disclosed in "Lexikon der Hilfsstoffe fur Pharmazie, Kosmetik und angrenzende Gebiete", published by H.P. Fielder, 4th Edition, and "Handbook of Pharmaceutical Excipients", 3rd Edition, published by A.H. Kibbe, American Pharmaceutical Association, Washington, USA, and Pharmaceutical Press, London.

The term filler generally means substances which serve to form the body of the tablet in the case of tablets with small amounts of active agent (e.g. less than 60% by weight). This means that fillers "dilute" the active agent(s) in order to produce an adequate tablet compression mixture. The normal purpose of fillers therefore is to obtain a suitable tablet size. Examples of preferred fillers are lactose, lactose derivatives, starch, starch derivatives, treated starch, chitin, cellulose and derivatives thereof, calcium phosphate, calcium hydrogen phosphate, sucrose, calcium carbonate, magnesium carbonate, magnesium oxide, maltodextrin, calcium sulphate, dextrates, dextrin and/or dextrose and hydrogenated vegetable oil. Fillers can be present in an amount of 0 to 80% by weight, preferably in an amount of 10 to 60% by weight based on the total weight of the composition.

A binder is generally a substance which is capable of increasing the strength of the resulting dosage form, especially the resulting tablets. Suitable binders are for example polyvinylpyrrolidone, hydroxypropyl cellulose, hydroxypropyl methylcellulose, methylcellulose, hydroxyethyl cellulose, sugars, dextran or corn starch. Binders can be present in an amount of 0 to 30% by weight, preferably in an amount of 2 to 15% by weight based on the total weight of the composition.

Glidants can be used to improve the flowability. Suitable glidants are for example alkaline earth metal salts of fatty acids, like stearic acid. The glidant can be present for example in an amount of 0 to 2% by weight, preferably in an amount of 0.5 to 1.5% by weight based on the total weight of the composition.

Disintegrants are compounds which enhance the ability of the dosage form, preferably the ability of the tablet, to break into smaller fragments when in contact with a liquid, preferably water. Suitable disintegrants are for example croscarmellose sodium, sodium carboxymethyl starch, cross-linked polyvinylpyrrolidone (crospovidone), sodium carboxymethylglycolate and sodium bicarbonate. The disintegrant can be present in an amount of 0 to 20% by weight, preferably in an amount of 1 to 15% by weight based on the total weight of the composition.

A suitable flow regulating agent is for example colloidal silica. The flow regulating agent can be present in an amount of 0 to 8% by weight, preferably in an amount of 0.1 to 3% by weight based on the total weight of the composition.

A suitable release agent is for example talcum. The release agent can be present in an amount of 0 to 5% by weight, preferably in an amount of 0.5 to 3% by weight based on the total weight of the composition.

The parmaceutical composition can preferably be further processed into an oral doasage form, such as a capsule or tablet.

The oral dosage form, preferably a tablet or a capsule, more preferably a tablet, can preferably be coated, preferably film coated.

In the present invention the following three types of film coatings are possible:
- film coatings without affecting the release of the active ingredient,
- gastric juice-resistant film coatings,
- retard film coatings.

Generally, film coatings can be prepared by using film-forming agents, such as waxes, cellulose derivatives, poly(meth)acrylate, polyvinylpyrrolidone, polyvinyl acetate phthalate, and/or shellac or natural rubbers, such as carrageenan.

It is preferred that the present tablet is coated with a gastric juice-resistant film coating. Alternatively, a capsule comprising a gastric juice-resistant film coating can be used.

The gastric juice-resistant film coating preferably is a film coating being stable in the pH range of about 0.7 to 3.0, which is supposed to be the pH value of human gastric juice found in the stomach. However, in an environment with a pH value of 5 to 9, which is supposed to be present in the (small) intestine of the human body, the gastric juice-resistant film coating preferably dissolves and the drug can be released.

The gastric juice-resistant film coating (often also referred to as enteric coating) can comprise film-forming agents, for example fats, fatty acids, waxes, alginates, shellac, polyvinyl acetate phthalate, cellulose derivatives such as carboxy methyl ethyl cellulose, cellulose acetate succinate, cellulose acetate phthalate, hydroxypropyl methyl cellulose phthalate, hydroxypropyl methyl cellulose acetate succinate, cellulose acetate trimellitate, and meth(acrylic)acid copolymers, such as methyl acrylate-methacrylic acid copolymers, methyl methacrylate-methacrylic acid copolymers and Eudragits (for example Eudragit^{®} L30D, Eudragit^{®} L, Eudragit^{®} S).

The coating is preferably free of active ingredient. It is further preferred that the thickness of the coating is 10 µm to 2 mm, preferably 50 to 500 µm.

The preferred coating may comprise a film-forming agent and one or more of the following: lubricant, surfactant, glidant, pigment and water.

The preferred coating according to an embodiment of the present invention can comprise, along with the film-forming agent, e.g. stearic acid as lubricant for plasticizing and dissolving the polymer, sodium lauryl sulfate as a surfactant for wetting and dispersing, talc as glidant, iron oxide yellow and/or titanium oxide as pigment(s) and optionally purified water.

The present pharmaceutical composition and/or the oral dosage form can be prepared by the methods well-known to a person skilled in the art, such as dry and wet granulation and direct compression.

Thus, the invention further relates to a method for the preparation of a phamraceutical composition as defined in claim 10.

Furhtermore, the invention relates to a phamraceutical composition as defined in claim 11.

In a preferred embodiment, the pharmaceutical composition and/or the oral dosage form can be administered one to three times a day, preferably once or twice a day, more preferably once a day.

Further described is crystalline tapentadol phosphate for use in the treatment of pain, preferably for use in the treatment of musculoskeletal pain.

### Experimental Part

### Analytical Methods

### X-ray Powder Diffraction (XRPD)

The samples were measured on a D8 Advance powder X-ray diffractometer (Bruker AXS, Karlsruhe, Germany) in a rotating PMMA sample holder (diameter: 25 mm; depth: 1 mm) in reflection mode (Bragg-Brentano geometry). Conditions of the measurements are summarized in the table below. Raw data were analyzed with the program EVA (Bruker AXS, Karlsruhe, Germany). Background subtraction and K*α*₂ stripping were not performed for the depicted diffractograms. Peak intensities were determined after background subtraction.

Conditions for powder diffraction measurements were as follows:

| | |
|---|---|
| Radiation | Cu K*α*₁/*α*₂ |
| Source | 34 kV / 40 mA |
| Detector | Vantec-1 (electronic window: 3°) |
| Kβ filter | Ni (diffracted beam) |
| measuring circle diameter | 435 mm |
| detector window slit | 12 mm |
| anti-scatter slit (diffracted beam) | 8 mm |
| divergence slit | v6.00 (variable) |
| Soller slit (incident /diffracted beam) | 2.5° |
| 2θ range | 2° ≤ 2θ ≤ 55° |
| step size | 0.016 |
| step time | 0.2 s |

### Dynamic Vapor Sorption

Vapor sorption experiments were performed in the instrument SPSx-1µ (Projekt Messtechnik, Ulm, Germany) at 25°C and the humidity cycles as shown below.

| Cycle no. | rel. humidity (% RH) | | Number of steps | Time (h) | Comments |
|---|---|---|---|---|---|
| | start value | end value | | | |
| 1 | 40 | 0 | 4 | | |
| 2 | 0 | 5 | 1 | | |
| 3 | 5 | 75 | 7 | | |
| 4 | 75 | 75 | 0 | 24 h | To investigate the absorption of water at the upper humidity level during stress conditions |
| 5 | 75 | 95 | 2 | | |
| 6 | 95 | 90 | 1 | | |
| 7 | 90 | 0 | 9 | | |
| 8 | 0 | 5 | 1 | | |
| 9 | 5 | 35 | 3 | | |

### Thermogravimetry (TGA)

The samples were placed in open aluminum crucibles (40 µL). The measured weight curve is displayed as a function of the program temperature.

| | |
|---|---|
| Apparatus: | Mettler-Toledo TGA/DSC1 (Mettler-Toledo GmbH, Gießen, Germany) |
| Aluminium crucible: | 40 µL (open) |
| Temperature range: | 25°C to 400°C |
| Heating rate: | 10°C / min |
| Nitrogen flow: | 50 mL / min |
| Software: | STARe Version 11.00 |

### Examples:

### Reference Example 1:

### Preparation of tapentadol phosphate according to conventional methods:

### Reference Example 1a:

400 mg (2.1 mmol) tapentadol base were dissolved in 20 ml isopropanol and the solution was stirred at 23°C. After addition of 140 µl 85% phosphoric acid (2.1 mmol), the formation of a white precipitate was observed. Upon filtration of the suspension, the isolated solid spontaneously liquefied.

### Reference Example 1b:

530 mg Tapentadol base were dissolved in 10 ml isopropanol and the solution was stirred at 23°C. Upon addition of 160 µl 85% phosphoric acid, a white solid precipitated. 20 ml n-pentane were added and the suspension was filtered. The isolated solid liquefied within 2 min.

### Reference Example 1c:

150 µl 85% phosphoric acid (2.3 mmol) were dissolved in 4 ml water. 0.5 g (2.3 mmol) solid tapentadol base were added and the suspension was warmed to 50°C for 5 min until a clear solution was obtained. The solution was cooled to RT, then frozen in a bath of liquid nitrogen and lyophilized. Within two minutes after removal of the flask from the freeze dryer, the white solid liquefied.

### Example 1:

### Preparation of crystalline Tapentadol phosphate with phosphoric acid:

In a 3 ml ball mill container, equipped with three 3 mm ZrO₂ balls, 200 mg Tapentadol base (Form B) and variable amounts of 85% phosphoric acid were mixed and milled for 15 min in the presence of 50 µl water. The products were isolated and analyzed by means of x-ray powder diffraction (XRPD).

The results are summarized below.

| **Exp.** | **H₃PO₄ 85%** | **Molar ratio (API / H₃PO₄)** | **water** | **result** |
|---|---|---|---|---|
| 1A | 61 µl | 1 : 1 | 50 µl | Crystalline tapentadol phosphate |
| 1B | 70 µl | 1: 1.15 | 50 µl | Crystalline tapentadol phosphate |

### Example 1':

Upscale preparation of crystalline tapentadol phosphate with phosphoric acid:
In a 20 ml ball mill container, equipped with twelve 3 mm ZrO₂ balls, 1 g tapentadol base (Form B) and 305 µl 85% phosphoric acid were mixed and milled for 15 min in the presence or absence of 250 µl water. The product was isolated and analyzed by means of x-ray powder diffraction (XRPD).

The result is summarized below.

| **Exp.** | **H₃PO₄ 85%** | **Molar ratio (Tap / H₃PO₄)** | **water** | **Result** |
|---|---|---|---|---|
| 1' | 305 µl | 1:1 | 250 µl | Crystalline tapentadol phosphate |

### Example 2:

### Preparation of crystalline tapentadol phosphate with sodium phosphate powder:

Tapentadol base (Form B) and Na(H₂PO₄)₂×2H₂O were given into a ball mill container together with three 3 mm ZrO₂ balls and water. These mixtures were treated in the ball mill for 15 min. The product was isolated and analyzed by means of x-ray powder diffraction (XRPD).

The result is summarized below.

| Exp. | Tapentadol base [mg] | NaH₂PO₄×2H₂O [mg] | Molar ratio | water | Result (XRPD) |
|---|---|---|---|---|---|
| 2 | 62.5 | 88.5 | 1:2 | 50 µl | Crystalline tapentadol phosphate |

### Example 3.1:

### Preparation of crystalline tapentadol phosphate (type A)

Tapentadol (1000 mg, 4.5 mmol) was dissolved at RT in a mixture of abs. ethanol (7.0 mL) and water (0.1 mL). Phosphoric acid 85% (0.31 mL; 4.5 mmol) was added under magnetic stirring at 23°C. The solution remained clear. After 15 minutes seed crystals of tapentadol phosphate were added and the mixture was stirred magnetically (200 rpm) at 23°C. Within one minute the crystallization started and after 10 min the formed suspension became too thick for stirring. It was stored at 23°C for 30 min. Cold (5°C) ethanol (20 mL) was added and the formed crystals were isolated immediately by vacuum filtration. The product was dried one hour in open atmosphere at 23°C (yield 976 mg).

| | |
|---|---|
| XRPD: | as shown in Figure 1 |

### Example 3.2:

### Preparation of crystalline tapentadol phosphate

Tapentadol (1500 mg, 6.8 mmol) was dissolved at RT in abs. ethanol (10.5 mL). Phosphoric acid 85% (0.464 mL; 6.8 mmol) was added under magnetic stirring at 23°C. The solution remained clear. After 15 minutes seed crystals of tapentadol phosphate (7 mg) were added and the mixture was stirred magnetically (200 rpm) at 23°C. Within 10 minutes crystallization started and after 1h a thick suspension had been formed. It was stored at 23°C for 30 min. Cold (5-10°C) ethanol (20 mL) was added and the formed crystals were isolated immediately by vacuum filtration. The product was dried in open atmosphere at 23°C overnight. Yield: 550 mg. Weight loss up to 150°C (TGA): 2.3%.

### Example 3.3:

### Preparation of crystalline tapentadol phosphate

Tapentadol (1000 mg, 4.5 mmol) was dissolved at RT in ethanol 96% v/v (7.0 mL). Phosphoric acid 85% (0.310 mL; 4.5 mmol) was added under magnetic stirring at 23°C. The solution remained clear. After 15 minutes seed crystals of tapentadol phosphate (5 mg) were added and the mixture was stirred magnetically (250 rpm) at 23°C. Within 5 minutes crystallization started and after 1 hour a thick suspension had been formed. It was stored at 23°C for 30 min. Cold (5-10°C) ethanol (20 mL) was added and the formed crystals were isolated immediately by vacuum filtration. The product was dried at 50°C / 9 mbar overnight. Yield: 933 mg. Weight loss up to 150°C (TGA): 1.7%.

### Example 3.4:

### Preparation of crystalline tapentadol phosphate

Tapentadol (2.51 g) was dissolved at RT in a mixture of abs. ethanol (17.5 mL) and water (0.25 mL). Phosphoric acid 85% (0.773 mL) was added under magnetic stirring at 23°C. The solution remained clear. After 15 minutes seed crystals of tapentadol phosphate (12 mg) were added and the mixture was stirred magnetically (200 rpm) at 23°C. Within one minute crystallization started and after 10 min the formed suspension became too thick for stirring. It was stored at 23°C for 50 min. Cold (5°C) ethanol (32 mL) was added and the formed crystals were isolated immediately by vacuum filtration. The product was dried 16 h at 50°C / 9 mbar (yield 2.54 g). Weight loss up to 150°C (TGA): 2.6%.

### Example 3.5:

### Preparation of crystalline tapentadol phosphate

Tapentadol (15.01 g) was dissolved at RT in a mixture of abs. ethanol (105 mL) and water (1.5 mL) and then heated at 50°C. Phosphoric acid 85% (0.55 mL) was added under magnetic stirring at 50°C. Seed crystals of tapentadol phosphate (0.75 g) were added, then additional phosphoric acid 85% (4.15 mL) was added dropwise at 50°C within 20 min. Crystallization started and the mixture was stirred 1 h at 50°C. It was cooled to 25°C within 45 min, then to 22°C. After stirring for 15 min at 22°C the product was isolated by vacuum filtration, washed with ethanol (40 mL) and dried at 50°C / 9 mbar for 16 h (yield 18.44 g). Weight loss up to 150°C (TGA): 2.0%.

### Example 3.6:

### Preparation of crystalline tapentadol phosphate

Tapentadol (50.04 g) was dissolved at RT in a mixture of abs. ethanol (350 mL) and water (5 mL) and then heated at 50°C. Phosphoric acid 85% (1.75 mL) was added under magnetic stirring at 50°C. Seed crystals of tapentadol phosphate (1.5 g) were added, then additional phosphoric acid 85% (13.9 mL) was added dropwise at 50°C. The mixture was stirred 1 h at 50°C. Additional phosphoric acid 85% (0.75 mL) was added and the mixture was stirred overnight at 50°C. It was then cooled to 22°C within 60 min. After stirring for 2 h at 22°C the product was isolated by vacuum filtration, washed with ethanol (170 mL) and dried at 50°C / 9 mbar for 16 h (yield 49.50 g). Weight loss up to 150°C (TGA): 1.7%.

### Example 3.7:

### Preparation of crystalline tapentadol phosphate

Tapentadol (446.4 g) was dissolved at RT in a mixture of abs. ethanol (3100 mL) and water (45 mL) and then heated at 50°C. Phosphoric acid 85% (15.8 mL) was added under stirring at 50°C. Seed crystals of tapentadol phosphate (7.8 g) were added, then additional phosphoric acid 85% (123 mL) was added dropwise at 50°C. The mixture was stirred overnight at 50°C. It was cooled to 22°C within 90 min. After stirring for 4 h at 22°C the product was isolated by vacuum filtration, washed with ethanol (1300 mL) and dried at 50°C / 9 mbar for 16 h (yield 485.1 g). Weight loss (TGA) up to 150°C: 2.6%.

### Example 3.8:

### Preparation of crystalline tapentadol phosphate

Tapentadol (505.0 g) was dissolved at RT in a mixture of abs. ethanol (3530 mL) and water (50 mL) and then heated at 50°C. Phosphoric acid 85% (17.8 mL) was added under stirring at 50°C. Seed crystals of tapentadol phosphate (7.9 g) were added, then additional phosphoric acid 85% (138.5 mL) was added dropwise at 50°C. The mixture was stirred overnight at 50°C. It was cooled to 22°C within 60 min. After stirring for 4 h at 22°C the product was isolated by vacuum filtration, washed with ethanol (1400 mL) and dried at 50°C / 9 mbar for 16 h (yield 524.2 g). Weight loss up to 150°C (TGA): 2.4%.

### Example 3.9:

### Preparation of crystalline tapentadol phosphate

Tapentadol (504.9 g) was dissolved at RT in a mixture of abs. ethanol (3520 mL) and water (50 mL). Phosphoric acid 85% (156 mL) was added within 10 min under stirring. The solution remained clear. Crystallization started after 20 min and the mixture became almost immobile after 45 min. It was slowly stirred for 3 h at RT. The suspension was diluted with cold (5 °C) ethanol (6700 mL) and the solid was isolated immediately by vacuum filtration. The product was dried at 50°C / 9 mbar for 16 h (yield 500.4 g). Weight loss up to 150°C (TGA): 2.2%.

### Example 3.10:

### Preparation of crystalline tapentadol phosphate

Tapentadol (468.5 g) was dissolved at RT in a mixture of abs. ethanol (3200 mL) and water (47 mL). Phosphoric acid 85% (145 mL) was added within 10 min under stirring. The solution remained clear. Crystallization started after 20 min and the mixture became almost immobile after 45min. It was slowly stirred for 3h at RT. The suspension was diluted with cold (5 °C) ethanol (6000 mL) and the solid was isolated immediately by vacuum filtration. The product was dried at 50°C / 9 mbar for 16 h (yield 484.1 g). Weight loss up to 150°C (TGA): 2.5%.

### Example 3.11:

### Preparation of crystalline tapentadol phosphate (type B)

200 mg of crystalline tapentadol phosphate type A obtained from Example 3.1 were heated at 70°C / 8 mbar for 12 h and tapentadol phosphate type B was obtained.

### Example 3.12:

### Preparation of crystalline tapentadol phosphate (type B)

Tapentadol (10.00 g, type A) was stirred dissolved as a suspension in isopropanol (150 mL) at 70°C for 5 hours. The solid was isolated from the still warm suspension by vacuum filtration and dried at 23°C / 8 mbar for one hour (yield 8.80g). The product consisted of type B.

The table below shows selected X-ray powder diffractions peaks and water contents of examples 3.4 to 3.10

| Example | XRPD (I) | XRPD (II) | XRPD (III) | XRPD (IV) | XRPD (V) | Water content (%) |
|---|---|---|---|---|---|---|
| 3.4 | 5.1 | 14.4 | 17.6 | 18.3 | 21.0 | 2.6 |
| 3.5 | 5.2 | 14.5 | 17.8 | 18.5 | 21.2 | 2.0 |
| 3.6 | 5.2 | 14.5 | 17.7 | 18.5 | 21.1 | 1.7 |
| 3.7 | 5,.1 | 14.4 | 17.6 | 18.3 | 21.0 | 2.6 |
| 3.8 | 5.1 | 14.4 | 17.6 | 18.3 | 21.0 | 2.4 |
| 3.9 | 5.1 | 14.4 | 17.6 | 18.3 | 21.0 | 2.2 |
| 3.10 | 5.1 | 14.4 | 17.7 | 18.3 | 21.0 | 2.5 |

From this table it can be seen that crystalline tapentadol phosphate can be obtained with different contents of water.

### Example 4:

### Preparation of crystalline tapentadol phosphate with calcium phosphate powders (milling experiments):

Tapentadol base (Form B) and Ca(H₂PO₄)₂×H₂O was given into a ball mill container together with three 3 mm ZrO₂ balls and water. These mixtures were treated in the ball mill for 15 min. The products were isolated and analyzed by means of X-ray powder diffraction (XRPD). CaHPO₄×2H₂O was detected in all cases. Subsequently, the samples were subjected to a DVS cycle as described above. After a DVS treatment, additional crystalline reflections corresponding to tapentadol phosphate appeared.

| Exp. | Tapentadol base [mg] | Ca(H₂PO₄)₂×H₂O [mg] | Molar ratio | water | No. balls (size) / vol (container) | Result (XRPD) after DVS |
|---|---|---|---|---|---|---|
| 4A | 300.4 | 300 | 1.14:1 | 200 µl | 12 (3 mm) / 20 ml | crystalline tapentadol phosphate |
| 4B | 280.2 | 319.5 | 1.0:1 | 200 µl | 12 (3 mm) / 20 ml | crystalline tapentadol phosphate |
| 4C | 360.1 | 240 | 1.70:1 | 200 µl | 12 (3 mm) / 20 ml | crystalline tapentadol phosphate |

The XRPD measurements of the examples revealed that crystalline tapendatol phosphate as depicted in Figure 1 was achieved.

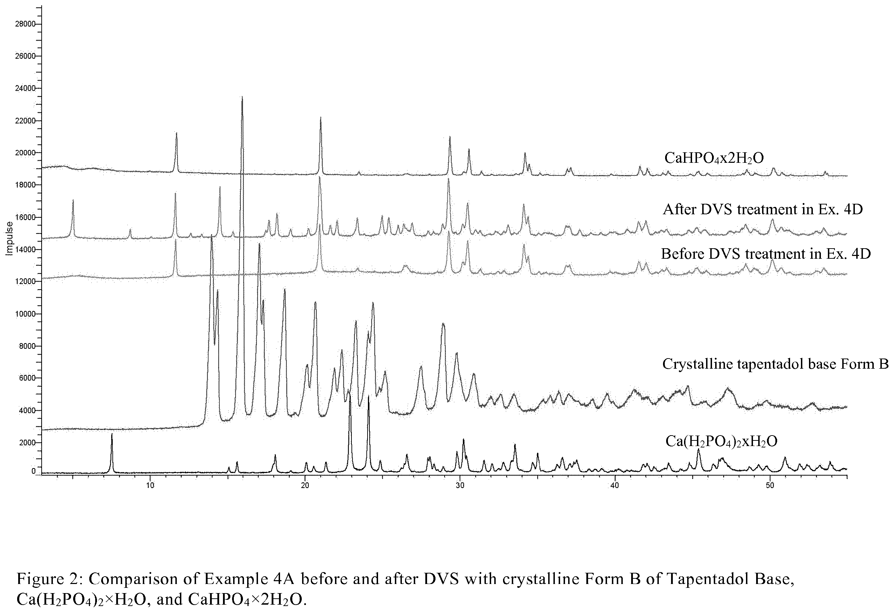

Further, it can be seen from Figure 2 that in Example 4 A after the step of subjecting the sample to a DVS the reflections of crystalline tapentadol phosphate appeared.

### Example 5:

### Characterization

### 5.1 Base titration of tapentadol phosphate

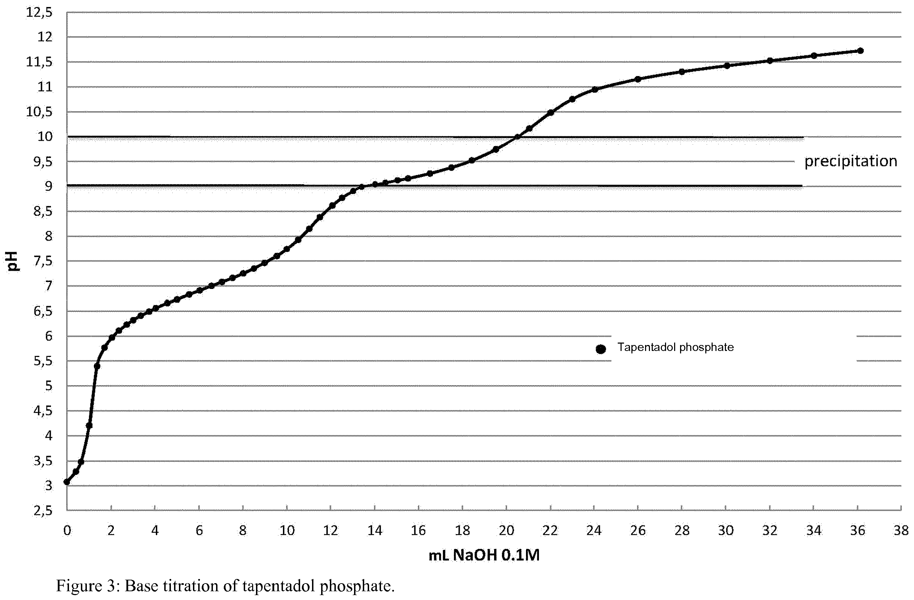

Tapentadol phosphate (300 mg) was dissolved in deionized water (40 mL). The solution was titrated at 23°C with 0.1M aqueous sodium hydroxide solution. The result is shown in Figure 3. The titration curve of tapentadol phosphate features three steps, corresponding to deprotonation of (H₂PO₄)⁻, (TAP-H)⁺ and (HPO₄)²⁻. Between pH 9 and pH 10 precipitation of tapentadol base is observed.

### 5.2. Water content

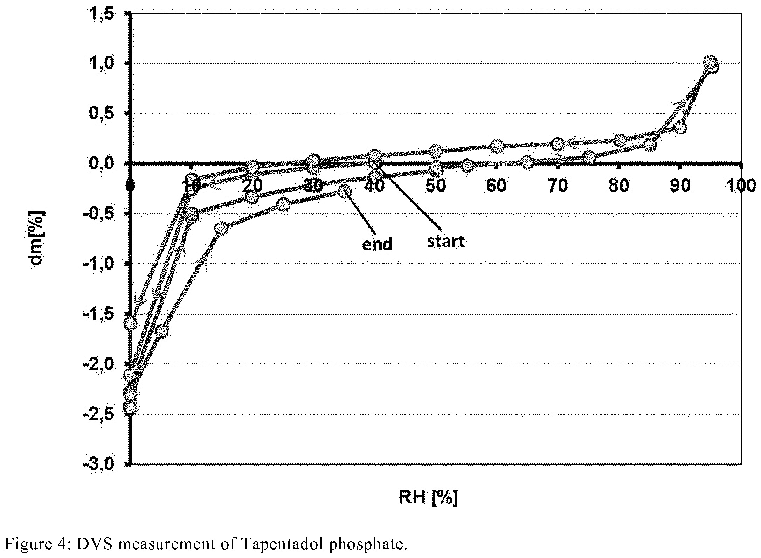

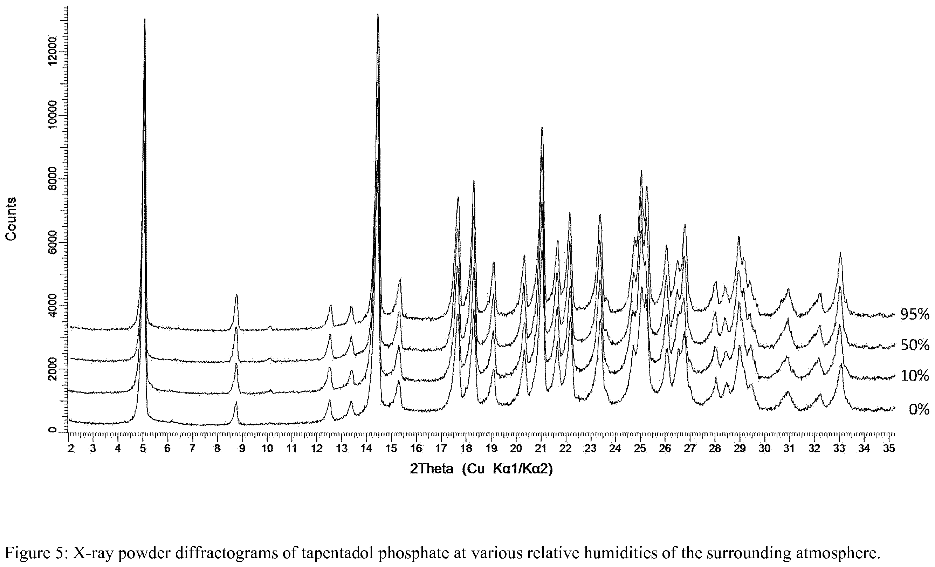

Tapentadol phosphate can contain water. TGA measurement indicated a water content of 2.6% (weight loss up to 150°C) for a sample prepared according to Example 3.1 which had been further dried at 30°C / 8 mbar for 1 day (type A). Results of a dynamic vapor sorption measurement of this sample are shown in Figure 4. There is no pronounced variation in water content between 20 and 80% relative humidity (RH). At 0% RH a maximal weight loss of 2.5% is observed, in reasonable agreement with the TGA result. The theoretical water content for the stoichiometry of tapentadol phosphate hemihydrate is 2.74%. No changes were observed in the X-ray powder diffractograms of the sample before and after DVS measurement. Diffractograms measured from samples which had been equilibrated at 0, 10, 50, and 95% RH are practically identical, with exception of the peak at 10.1° (2Θ), which becomes weaker upon lowering of the relative humidity (Figure 5).

### 5.3 Molecular structure of crystalline tapentadol phosphate

The crystal was measured on an Oxford Diffraction XCALIBUR2 diffractometer with area detector. The absolute configuration of the determined molecular structure matched the expected configuration; the Flack parameter was refined to a value of 0.10(12), thus it corroborates the assignment. Hydrogen atoms were refined according to a riding model (in case of the contained water molecules the hydrogen atoms were omitted). The structure is shown in Figure 7.

| | |
|---|---|
| Empirical formula | C₁₄H₂₆NO₆P |
| Formula weight | 335.34 |
| Temperature [K] | 150 |
| Wavelength [Å] | 0.71073 |
| Crystal system | hexagonal |
| Space group | *P*6₅ |
| Unit cell dimensions | |
| *a* [Å] | 20.2002(6) |
| *c* [Å] | 7.5599(2) |
| Volume [Å³] | 2671.52(7) |
| *Z* | 6 |
| Density (calculated) [g·cm⁻³] | 1.251 |
| Absorption coefficient [mm⁻¹] | 0.180 |
| *F*(000) | 1080 |
| Crystal size [mm] | 0.07 × 0.10 × 0.27 mm |
| Theta range for data collection [°] | 2.94 to 29.48 (reflection count complete at 25.05) |
| Index ranges | -26≤*h*≤27, -26≤*k*≤13, -10≤*l*≤7 |
| Reflections collected | 9740 |
| Independent reflections [*R*(int)] | 3713 (0.034) |
| Completeness to Theta = 25.05 | 98.9% |
| Absorption correction | Semi-empirical from equivalents |
| Max. and min. transmission | 0.88 and 1.00 |
| Refinement method | Full-matrix least-squares on *F*² |
| Data / parameters / restraints | 3712/222/8 |
| Goodness-of-fit on *F*² | 1.000 |
| Final R indices [*I*>2σ(*I*)] | *R*₁ = 0.0448, w*R*₂ = 0.0627 |
| Flack parameter | 0.10(12) |
| Final *R* indices [all data] | *R*₁ = 0.0529, w*R*₂ = 0.0663 |
| Largest diff. peak and hole [e·Å^{- 3}] | 0.36 and -0.35 |

## Claims

1. Use of crystalline tapentadol phosphate for preparing a pharmaceutical composition further comprising at least one pharmaceutically acceptable excipient.

2. Use according to claim 1, wherein the crystalline tapentadol phosphate has a water content of 0.5 to 4.0 % determined by thermogravimetry.

3. Use according to claim 1 or 2, wherein the at least one pharmaceutically acceptable excipient comprises fillers, binders, glidants, disintegrants, lubricants, flow regulating agents and release agents.

4. Use according to any one of claims 1 to 3, wherein the filler is present in an amount of 10 to 60% by weight based on the total weight of the composition.

5. Use according to any one of claims 1 to 4, wherein the binder is present in an amount of 2 to 15% by weight based on the total weight of the composition.

6. Use according to any one of claims 1 to 5, wherein the glidant is present in an amount of 0.5 to 1.5% by weight based on the total weight of the composition.

7. Use according to any one of claims 1 to 6, wherein the disintegrant is present in an amount of 1 to 15% by weight based on the total weight of the composition.

8. Use according to any one of claims 1 to 7, wherein the flow regulating agent is present in an amount of 0.1 to 3% by weight based on the total weight of the composition.

9. Use according to any one of claims 1 to 8, wherein the release agent is present in an amount of 0.5 to 3% by weight based on the total weight of the composition.

10. Method for the preparation of a pharmaceutical composition comprising dry granulation, wet granulation or direct compression of crystalline tapentadol phosphate and at least one pharmaceutically acceptable excipient.

11. Pharmaceutical composition obtainable by dry granulation, wet granulation or direct compression of crystalline tapentadol phosphate and at least one pharmaceutically acceptable excipient.

## Patentansprüche

1. Verwendung von kristallinem Tapentadolphosphat zum Zubereiten einer pharmazeutischen Zusammensetzung, die ferner mindestens einen pharmazeutisch verträglichen Hilfsstoff umfasst.

2. Verwendung nach Anspruch 1, wobei das kristalline Tapentadolphosphat einen durch Thermogravimetrie bestimmten Wassergehalt von 0,5 bis 4,0 % aufweist.

3. Verwendung nach Anspruch 1 oder 2, wobei der mindestens eine pharmazeutisch verträgliche Hilfsstoff Füllstoffe, Bindemittel, Gleitmittel, Zerfallsmittel, Schmiermittel, Fließregulierungsmittel und Trennmittel umfasst.

4. Verwendung nach einem der Ansprüche 1 bis 3, wobei der Füllstoff in einer Menge von 10 bis 60 Gewichts-% auf der Basis des Gesamtgewichts der Zusammensetzung vorhanden ist.

5. Verwendung nach einem der Ansprüche 1 bis 4, wobei das Bindemittel in einer Menge von 2 bis 15 Gewichts-% auf der Basis des Gesamtgewichts der Zusammensetzung vorhanden ist.

6. Verwendung nach einem der Ansprüche 1 bis 5, wobei das Gleitmittel in einer Menge von 0,5 bis 1,5 Gewichts-% auf der Basis des Gesamtgewichts der Zusammensetzung vorhanden ist.

7. Verwendung nach einem der Ansprüche 1 bis 6, wobei das Zerfallsmittel in einer Menge von 1 bis 15 Gewichts-% auf der Basis des Gesamtgewichts der Zusammensetzung vorhanden ist.

8. Verwendung nach einem der Ansprüche 1 bis 7, wobei das Fließregulierungsmittel in einer Menge von 0,1 bis 3 Gewichts-% auf der Basis des Gesamtgewichts der Zusammensetzung vorhanden ist.

9. Verwendung nach einem der Ansprüche 1 bis 8, wobei das Trennmittel in einer Menge von 0,5 bis 3 Gewichts-% auf der Basis des Gesamtgewichts der Zusammensetzung vorhanden ist.

10. Verfahren für die Zubereitung einer pharmazeutischen Zusammensetzung, das Trockengranulierung, Nassgranulierung oder direkte Kompression von kristallinem Tapentadolphosphat und mindestens eines pharmazeutisch verträglichen Hilfsstoffs umfasst.

11. Pharmazeutische Zusammensetzung, die durch Trockengranulierung, Nassgranulierung oder direkte Kompression von kristallinem Tapentadolphosphat und mindestens eines pharmazeutisch verträglichen Hilfsstoffs erhalten werden kann.

## Revendications

1. Utilisation de tapentadol phosphate cristallin pour préparer une composition pharmaceutique comportant en outre au moins un excipient pharmaceutiquement acceptable.

2. Utilisation selon la revendication 1, dans laquelle le tapentadol phosphate cristallin a une teneur en eau de 0,5 à 4,0 % déterminée par thermogravimétrie.

3. Utilisation selon la revendication 1 ou 2, dans laquelle le au moins un excipient pharmaceutiquement acceptable comporte des charges, des liants, des agents glissants, des délitants, des lubrifiants, des agents de régulation d'écoulement et des agents de libération.

4. Utilisation selon l'une quelconque des revendications 1 à 3, dans laquelle la charge est présente en une quantité de 10 à 60 % en poids basé sur le poids total de la composition.

5. Utilisation selon l'une quelconque des revendications 1 à 4, dans laquelle le liant est présent en une quantité de 2 à 15 % en poids basé sur le poids total de la composition.

6. Utilisation selon l'une quelconque des revendications 1 à 5, dans laquelle l'agent glissant est présent en une quantité de 0,5 à 1,5 % en poids basé sur le poids total de la composition.

7. Utilisation selon l'une quelconque des revendications 1 à 6, dans laquelle le délitant est présent en une quantité de 1 à 15 % en poids basé sur le poids total de la composition.

8. Utilisation selon l'une quelconque des revendications 1 à 7, dans laquelle l'agent de régulation d'écoulement est présent en une quantité de 0,1 à 3 % en poids basé sur le poids total de la composition.

9. Utilisation selon l'une quelconque des revendications 1 à 8, dans laquelle l'agent de libération est présent en une quantité de 0,5 à 3 % en poids basé sur le poids total de la composition.

10. Procédé pour la préparation d'une composition pharmaceutique comportant une granulation par voie sèche, une granulation par voie humide ou une compression directe du tapentadol phosphate cristallin et d'au moins un excipient pharmaceutiquement acceptable.

11. Composition pharmaceutique pouvant être obtenue par une granulation par voie sèche, une granulation par voie humide ou une compression directe du tapentadol phosphate cristallin et d'au moins un excipient pharmaceutiquement acceptable.
